# EUROPÄISCHE PATENTSCHRIFT

(11) **EP 1 645 625 B1**
(45) Veröffentlichungstag und Bekanntmachung des Hinweises auf die Patenterteilung: **09.03.2011**
(21) Anmeldenummer: 05090257.6
(22) Anmeldetag: 14.09.2005
(51) Int. Cl.: C12N 5/071, C12M 3/06

(54) **Polymeres Substrat zur Kultivierung von Zellen, insbesondere von Keratinozyten**
Polymer Substrat for Cell Cultivation, Especially Keratinocyte Cultivation
Support polymère pour la culture de cellules, en particulier de kératinocytes

(30) Priorität: 08.10.2004 DE 102004049758
(43) Veröffentlichungstag der Anmeldung: 12.04.2006
(73) Patentinhaber: GKSS-Forschungszentrum Geesthacht GmbH, 21502 Geesthacht (DE)
(72) Erfinder: Boese, Gregor, Dr., 12047 Berlin (DE); Albrecht, Wolfgang, Dr., 14513 Teltow (DE); Malsch, Günther, Dr., 14513 Teltow (DE); Groth, Thomas, Dr., 10439 Berlin (DE); Lendlein, Andreas, Prof. Dr., 14167 Berlin (DE); Stark, Hans-Jürgen, 74909 Meckesheim (DE); Fusenig, Norbert, Prof. Dr., 69118 Heidelberg (DE)
(74) Vertreter: Reinstädler, Diane

(56) Entgegenhaltungen:
- EP-A- 0 476 875
- WO-A-88/05688
- US-A- 4 477 634
- GROTH THOMAS ET AL: "Interaction of human skin fibroblasts with moderate wettable polyacrylonitrile-copolymer membranes" JOURNAL OF BIOMEDICAL MATERIALS RESEARCH, Bd. 61, Nr. 2, August 2002 (2002-08), Seiten 290-300, XP002400025 ISSN: 0021-9304
- KRASTEVA N ET AL: "Membranes for biohybrid liver support systems-investigations on hepatocyte attachment, morphology and growth" BIOMATERIALS, ELSEVIER SCIENCE PUBLISHERS BV., BARKING, GB, Bd. 23, Nr. 12, Juni 2002 (2002-06), Seiten 2467-2478, XP004348019 ISSN: 0142-9612
- ALBRECHT W ET AL: "Amination of polyimides: A new wet-chemical route for surface functionalization." INTERNATIONAL JOURNAL OF ARTIFICIAL ORGANS, Bd. 26, Nr. 7, Juli 2003 (2003-07), Seite 640, XP002404428 & XXX CONGRESS OF THE EUROPEAN SOCIETY FOR ARTIFICIAL ORGANS (ESAO) ON HIGH TECH AND MEDICINE; AACHEN, GERMANY; SEPTEMBER 03-06, 2003 ISSN: 0391-3988
- TRIMPERT CHRISTIANE ET AL: "Poly(ether imide) membranes modified with poly(ethylene imine) as potential carriers for epidermal substitutes." MACROMOLECULAR BIOSCIENCE. 12 APR 2006, Bd. 6, Nr. 4, 12. April 2006 (2006-04-12), Seiten 274-284, XP002400027 ISSN: 1616-5187
- MCALPINE J B ET AL: "NEW ANTIBIOTICS FROM GENETICALLY ENGINEERED ACTINOMYCETES I. 2-NORERYTHROMYCINS, ISOLATION AND STRUCTURAL DETERMINATIONS" JOURNAL OF ANTIBIOTICS, JAPAN ANTIBIOTICS RESEARCH ASSOCIATION, TOKYO, JP, vol. 40, no. 8, 1 January 1987 (1987-01-01), pages 1115-1122, XP008055116 ISSN: 0021-8820
- DONADIO S ET AL: "MODULAR ORGANIZATION OF GENES REQUIRED FOR COMPLEX POLYKETIDE BIOSYNTHESIS" SCIENCE, AMERICAN ASSOCIATION FOR THE ADVANCEMENT OF SCIENCE, WASHINGTON, DC; US LNKD- DOI:10.1126/SCIENCE.2024119, vol. 252, 3 May 1991 (1991-05-03), pages 675-679, XP002035890 ISSN: 0036-8075
- HAYDOCK STEPHEN F ET AL: "Divergent sequence motifs correlated with the substrate specificity of (methyl)malonyl-CoA:acyl carrier protein transacylase domains in modular polyketide synthases" FEBS LETTERS, ELSEVIER, AMSTERDAM, NL LNKD- DOI:10.1016/0014-5793(95)01119-Y, vol. 374, no. 2, 1 January 1995 (1995-01-01), pages 246-248, XP002194846 ISSN: 0014-5793

## Beschreibung

Die Erfindung betrifft ein polymeres Substrat sowie seine Verwendung zur Kultivierung von Zellen, insbesondere von Keratinozyten. Die Erfindung betrifft ferner eine organotypische Hybridstruktur umfassend ein polymeres Substrat und darauf kultivierte Zellen sowie ein Verfahren zur Erzeugung einer Gewebestruktur, insbesondere einer epidermalen Hautstruktur.

Die Therapie von chronischen Gewebedefekten, insbesondere diabetischen, venösen oder druckinduzierten Ulzerationen, sowie großflächiger Verbrennungswunden stellt ein beträchtliches medizinisches und wirtschaftliches Problem dar. So leiden - bei steigender Inzidenz - etwa 2 % der Weltbevölkerung an schwer heilenden Wunden bei erheblichen durchschnittlichen, Behandlungskosten. Die Behandlung von chronischen Gewebedefekten und Wunden durch Tissue Engineering (TE), das heißt mit künstlich kultivierten Gewebestrukturen, zeigt erste klinische Erfolge. Ein weiterer wichtiger Anwendungsbereich von *in vitro* kultivierten Hautsubstituten stellt ihre Verwendung für dermatologische Testungen dar. Als Alternativen zu teuren klinischen Studien oder Tierversuchen können in durch Tissue Engineering erzeugten organotypischen Testmodellen Wundheilung, Alterung oder Hautirritationen, verursacht durch physikalische oder chemische Einflüsse, untersucht werden.

Rein biologische Hautersatzprodukte, wie Xenotransplantate, Leichenhaut und allogene vitale Spenderhaut, erlauben nur eine temporäre Abdeckung der Wunde und sollen die Neubildung einer dermalen Struktur durch Induktion eines Granulationsgewebes stimulieren. Jedoch besteht stets ein Infektionsrisiko und die Spenderhaut wird nach einiger Zeit wegen immunologischer Unverträglichkeit abgestoßen bzw. muss vor der Transplantation von Spalthaut (das heißt autologer Spenderhaut) entfernt werden.

Daneben sind hybride Hautersatzprodukte bekannt, die eine biologisch abgeleitete Komponente und ein synthetisches, stabiles Material enthalten, welches dem Verschluss und der Abdeckung der Wunde dient. Aus US 5,460,939 A ist beispielsweise eine dreidimensionale Trägerstruktur aus einem resorbierbaren oder nicht resorbierbaren Material zur Besiedelung mit Zellen auf einer semipermeablen Membran bekannt. Auf der Trägerstruktur werden Fibroblasten über mehrere Wochen *in vitro* kultiviert, um ein dreidimensionales Gewebenetzwerk zu erhalten. Hierunter fällt das Produkt "Biobrane" (Fa. Dow Hickam/Bertek Pharmaceuticals), das aus einem mit Schweinekollagen I beschichteten Nylongewebe besteht, welches an einer Silikonmembran gebunden ist. Damit soll die Adhäsion im Wundbett und das Einwachsen von Fibroblasten und Blutgefäßen verbessert werden. Ein bei der Behandlung von Verbrennungen verbreitetes Material ("Integra", Fa. Integra LifeScience Corp.) besteht aus einer der Abdeckung des Wundbettes dienenden Silikonmembran, auf deren Unterseite vernetztes Rinderkollagen und Glykosaminoglukane verankert sind, was die Regeneration dermaler Strukturen fördern soll (US 5,489,304 A). Trotz guter klinischer Erfahrungen werden zuweilen Infektionen beobachtet, die eine vorzeitige Entfernung des Materials erforderlich machen. Ein weiteres Produkt verwendet autologe Hautzellen, die als Suspension in einem Fibrinkleber eingebettet und als "Haut aus der Tube" auf das Wundbett aufgebracht werden. Diese Vorgehensweise ist für die Behandlung großflächiger tiefer Wunden weniger geeignet.

Darüber hinaus sind auch TE-Produkte mit meist allogenen Zell- beziehungsweise Gewebestrukturen bekannt. Zu den kommerziell erhältlichen TE-Produkten zählt "TransCyte" (Fa. Advanced Tissue Science Inc.) und besteht aus einem Nylonnetz mit daran immobilisierten neonatalen allogenen Fibroblasten, die während einer *in vitro* Kultivierung von mehr als zwei Wochen wachsen und dabei extrazelluläre Matrixkomponenten produzieren. Durch Kryokonservierung werden die Zellen abgetötet, so dass nur noch die extrazelluläre Matrix verbleibt. Diese wird nach Transplantation von Fibroblasten besiedelt und soll die Epithelisierung der Wunde beschleunigen. Ferner ist das zweischichtige allogene Produkt "Apligraf" bekannt (Fa. Organogenesis Inc.), das aus einem Gel aus Typ I Rinderkollagen mit lebenden neonatalen Fibroblasten mit einer darüber liegenden epidermalen Schicht neonataler Keratinozyten besteht. Dieses komplexe und teure Hautersatzprodukt wird vor allem zur Behandlung schwer heilender Ulcera eingesetzt und soll deren Heilung fördern. Dabei werden die allogenen Spenderzellen nach einiger Zeit infolge von Abstoßungsreaktionen beseitigt und durch autologe einwandernde Zellen ersetzt (Falanga V. et al., Arch Dermatol. 134(3), 1998, 293-300). Ein weiteres, unter dem Namen "Dermagraft" bekanntes Produkt umfasst eine kryokonservierte dermale Struktur, die aus neonatalen allogenen Fibroblasten besteht, die auf einem resorbierbaren Polymergerüst aus Polyglycogen kultiviert werden. Die Fibroblasten wachsen in vitro bis zur Konfluenz, wobei sie Wachstumsfaktoren und extrazelluläre Matrixkomponenten sezernieren und damit eine vitale Struktur ausbilden, die transplantiert werden kann (Hansbrough J.F. et al., Surgery 111(4), 1992, 438-446).

Aus Groth et al. (J. Biomed. Mat. Res., 61(2), 2002, 290-300) ist die Kultivierung von Fibroblasten auf Membranen bekannt, die aus einem Copolymer aus Acrylnitril (AN) und einem der Comonomeren N-Vinylpyrolidon (NVP), Natrium-2-methyl-2-propen-1-sulfonsäure (NaMAS) oder Aminoethylmethacrylat (NaMAS) besteht.

Krasteva et al. (Biomaterials, 23(12), 2002, 2467-2478) beschreiben die Kultivierung von Hepatozyten auf Membranen aus Polyetherimid (PEI), reinem Polyacrylnitril (PAN) oder P(AN-NVP) oder Polyvinylidendifluorid (PVDF).

In Albrecht et al. (Macromol. Chem. Phys. 204(3), 2003, 510-521) werden die Synthese aminierter PEI-Membranen beschrieben sowie ihre physikalischen Eigenschaften.

Die Kultivierung von Keratinozyten auf Polyurethanmembranen ist in Rennekampff et al. (Surgery, 120(16), 1996, 16-22) und Wright et al. (Burns 24, 1998, 7-17) beschrieben.

Insgesamt kann festgestellt werden, dass derzeit kein Produkt bekannt ist, das bei moderaten Kosten die Funktionen natürlicher Haut befriedigend erfüllt. Als zelluläres Material sind insbesondere allogene Zellen etabliert, die einen wesentlichen Grund für Abstoβungsreaktionen darstellen. Sofern nicht-resorbierbare Polymere verwendet werden, besteht die Notwendigkeit, diese wieder zu entfernen, was neben dem medizinischen Aufwand häufig mit der Auslösung von Blutungen verbunden ist. Dementsprechend liegt der vorliegenden Erfindung die Aufgabe zugrunde, ein Substrat zur Verfügung zu stellen, das einerseits eine geeignete Grundlage zur *in vitro* Kultivierung von Hautstrukturen darstellt und sich andererseits bei seiner Entfernung von der behandelten Wunde einfach von dieser ablösen lässt, ohne zu einer Verletzung der neugebildeten Hautstruktur zu führen.

Diese Aufgabe wird durch die Verwendung eines polymeren Substrats zur Kultivierung von Keratinozyten gelöst, wobei das Substrat in Form einer sauerstoffdurchlässigen Flachmembran aus einem nicht resorbierbaren Polymer vorliegt und die Membran einen Amingehalt von mindestens 10 nmol Aminfunktionen pro cm² Membran, aufweist und wobei die Polymersubstanz der Membran ein Copolymer aus Acrylnitril (AN) und 3-Aminopropylmethacrylamid (APMA) umfasst oder die Membran eine mit einem Polyethylenimin aminierte Polyetherimid-Membran ist. Es wurde nämlich gefunden, dass luftdurchlässige, aminierte Polymermaterialien in Form poröser Membranen ausgezeichnete Supportstrukturen für die Ansiedlung primärer Keratinozyten darstellen, wenn der Amingehalt des Materials entweder durch Modifizierung der Membransubstanz selbst oder der Membranoberfläche optimal eingestellt wird und die Porosität der Membran eine ausreichende Luftdurchlässigkeit gewährleistet. Das erfindungsgemäße Substrat zeichnet sich dadurch aus, dass es zunächst die Proliferation der Keratinozyten fördert und aufgrund einer relativ schwachen Adhäsion basaler Zellen das spätere Ablösen der Membran ohne Gewebezerstörung ermöglicht.

Diese vorteilhaften Eigenschaften werden besonders durch einen Amingehalt von mindestens 50 nmol/cm², insbesondere von 100 bis 550 nmol/cm², vorzugsweise von 200 bis 500 nmol/cm², besonders bevorzugt von etwa 350 bis 450 nmol Amin pro cm² Membran erzielt. Dabei ist unerheblich, ob dieser Amingehalt aus einer Substanz- oder eine Oberflächenmodifizierung resultiert. Im Falle der Substanzmodifizierung wird ein Polymer für die Membranherstellung verwendet, welches entsprechende Aminfunktionen aufweist (Substanzmodifikation i.e.S.) oder welchem eine Polymerkomponente mit entsprechenden Aminfunktionen zugemischt ist (Volumenmodifikation). Hingegen wird bei der Oberflächenmodifizierung die Membranoberfläche nachträglich mit Aminfunktionen ausgestattet, das heißt entweder eine kovalente Oberflächenfunktionalisierung mit aminhaltigen Gruppen (beispielsweise durch nasschemische Modifizierung oder Pfropfung) oder eine nicht kovalente Beschichtung der Membran mit einem aminhaltigen Material. Denkbar ist aber auch die Verwendung einer Membran, die sowohl substanz- als auch oberflächenaminiert ist. Dabei wird im Sinne der vorliegenden Erfindung unter dem flächenbezogenen Amingehalt der molare Gesamtgehalt an Aminfunktionen pro cm² ausgeschnittener Membran verstanden, und nicht nur die auf der Membranoberfläche vorliegenden frei zugänglichen Amingruppen. Die Aminierung ist mit einer Hydrophilisierung der Materialoberfläche verbunden, die durch Kontaktwinkelmessung charakterisiert werden kann.

Im Rahmen der vorliegenden Erfindung wird unter Aminfunktion jegliche stickstoffhaltige Funktion verstanden, in welcher der Stickstoff Bindungen zu Kohlenstoff und/oder Wasserstoff aufweist, insbesondere primäre, sekundäre und tertiäre Aminogruppen (R-NH₂, R¹-NH-R² bzw. NR¹R²R³), primäre und sekundäre Iminogruppen (R=NH bzw. R¹=N-R²), quartäre Ammoniumgruppen (⁺NR¹R²R³R⁴), primäre, sekundäre und tertiäre Amidgruppen (R-CO-NH₂, R¹-CO-NHR² bzw. R¹-CO-NR²R³) und Imidgruppen (R¹-CO-N=R²) und Mischungen von diesen, worin die Reste R, R¹, R² und R³ jeweils einen unverzweigten oder verzweigten, gesättigten oder ungesättigten, zyklischen oder azyklischen Kohlenwasserstoffrest bedeuten. Nach einer bevorzugten Ausgestaltung der Erfindung umfasst der Amingehalt insbesondere solche Stickstoffgruppen aus der genannten Gruppe, in denen der Stickstoff ein oder zwei Wasserstoffbindungen aufweist. Derart aminierte Substrate zeichnen sich gegenüber anderen durch eine wesentlich geringere Adhäsion der gebildeten Gewebestrukturen an der Membranoberfläche und somit durch ein einfacheres, zerstörungsfreies Ablösen aus. Vorzugsweise betreffen die oben angegebenen optimierten Amingehalte primäre und/oder sekundäre Aminogruppen (R-NH₂, R¹-NH-R²).

Erfindungsgemäß handelt es sich bei dem Substrat um eine durch Substanzmodifizierung i.e.S. aminierte Membran, die als Polymersubstanz ein Copolymer aus einem Acrylnitril (AN) und einem amingruppenhaltigen Comonomer enthält, nämlich 3-Aminopropylmethacrylamid (APMA) beziehungsweise sein Hydrochlorid-Addukt.

Ein erfindungsgemäßes Beispiel für eine Oberflächenfunktionalisierung stellt eine mit Polyethylenimin(en) aminierte Polyethermid-Membran (PEI-Membran) dar.

Neben dem beschriebenen Amingehalt zeichnet sich das erfindungsgemäße Substrat durch seine Porosität aus, die einerseits einen ausreichenden Gastransport und eine hinreichende Diffusion von Metaboliten durch die Membran gewährleistet und andererseits die zu versorgende Wunde vor Infektionen schützen soll. Dies wird durch Verwendung einer für Mikroorganismen, insbesondere Bakterien, undurchlässigen Ultrafiltrationsmembran erzielt. Diese Membran weist bevorzugt eine Porengröße von höchstens 0,5 µm, insbesondere höchstens 0,1 µm auf sowie eine Wasserdurchlässigkeit von mindestens 0,5 l/m²hPa, insbesondere von mindestens 1,0 l/m²hPa auf.

Für die Ansiedlung von Zellen, insbesondere Keratinozyten, hat es sich zudem als vorteilhaft erwiesen, wenn die Membran eine plastische Oberflächenstruktur aufweist, die beispielsweise durch nachträgliche Prägung der Membran oder bereits während des Herstellungsprozesses der Membran erzeugt werden kann. Eine solche Textur fördert nicht nur die Besiedelung der Oberfläche, sondern kann darüber hinaus auch die Bildung eines gerichteten Gewebewachstums induzieren, wodurch die natürlichen organomechanischen Eigenschaften der Haut besonders gut nachgebildet werden.

Erfindungsgemäß handelt es sich bei dem für die Membran verwendeten Polymer um ein biologisch, insbesondere enzymatisch nicht resorbierbares Material, das nach dem Transfer und der Ausbildung eines dichten epidermalen Zellrasens restlos von diesem gelöst und entfernt wird. Hierdurch werden Problematiken, die sich im Zusammenhang mit Abbauprodukten ergeben können, vermieden.

Die Erfindung betrifft insbesondere die Verwendung des vorstehend beschriebenen polymeren Substrats zur Erzeugung von epidermalen Hautstrukturen, die als Hauttransplantate oder als Hautsubstitute für dermatologische und pharmakologische Testreihen eingesetzt werden können. Im Falle der Erzeugung von epidermalen Hauttransplantaten werden bevorzugt dem Patienten entnommene autologe Zellen für die Kultivierung verwendet.

Ein weiterer Aspekt der Erfindung betrifft eine organotypische Hybridstruktur, die das oben beschriebene erfindungsgemäße polymere Substrat sowie auf dem Substrat kultivierte Keratinozyten beziehungsweise eine epidermale Hautstruktur umfasst.

Noch ein weiterer Aspekt betrifft ein Verfahren zur Erzeugung einer Gewebestruktur, insbesondere einer epidermalen Hautstruktur, mit den Schritten:
- Kultivierung von primären Zellen, insbesondere von Keratinozyten, über eine erste Kultivierungsdauer auf dem polymeren Substrat,
- Transfer des mit den Zellen besiedelten Substrats auf eine Zielstruktur, so dass das Substrat mit seiner zellzugewandten Seite auf der Zielstruktur aufliegt,
- Kultivierung der Zellen für eine zweite Kultivierungsdauer und
- Ablösung und Entfernen des Substrats von der gebildeten Gewebestruktur (unter Verbleib von dieser auf der Zielstruktur).

Mit anderen Worten wird das erfindungsgemäße Substrat verwendet, um zunächst Keratinozyten kurzzeitig in vitro anzuzüchten und diese dann als Epithelschicht auf eine Zielstruktur, beispielsweise auf ein Dermisäquivalent oder eine Wundfläche, aufzubringen. Dies erfolgt in der Weise, dass die Membran mit ihrer Zellseite mit der Zielstruktur (Dermisäquivalent, Wundfläche) in Kontakt kommt. Dabei erfolgt die erste Kultivierung vorzugsweise nur bis zu einem subkonfluenten Zustand der Zellen, das heißt ohne eine geschlossene Zellschicht auszubilden. Zellkulturversuche haben ergeben, dass die erfindungsgemäßen aminierten Flachmembranen eine schnelle Transmigration und Umpolarisierung (Umorientierung) der Keratinozyten erleichtern, wenn sie mit ihrer Oberseite auf eine Kulturoberfläche, beispielsweise auf Zellkulturschalen oder Kollagenmatrizes, aufgebracht werden. Dort haften sie an und bilden mehrschichtige Epithelien aus. Dabei führt die Ausbildung von Hornschichten an der Oberseite, das heißt der dem Substrat zugewandten Seite, zu einer spontanen Ablösung des Trägermaterials und damit seiner problemlosen Entfernung. Das Verfahren ist besonders geeignet, Keratinozyten großflächig auf Wunden aufzubringen, damit sie dort unter dem Schutz des Substratmaterials anwachsen können und dieses nach erfolgter Bedeckung des Zielsubstrates beziehungsweise der Wundfläche spontan abzustoßen. Bereits eine kleine Hautbiopsie oder wenige Zellen aus der Haarwurzel sind ausreichend, um innerhalb einer kurzen Zeit von wenigen Tagen einen nahezu geschlossenen Epithelzellrasen autologer Zellen zu erhalten. Die Ultrafiltrationsmembranen sind undurchlässig für Bakterien, gestatten jedoch den für eine zügige Stratifizierung notwendigen Gasaustausch bei Luftexposition der organotypischen Kultur. Die Flachmembranen verbleiben zunächst als Schutz sowie zur erleichterten Handhabung bis zur klinischen Applikation auf der Zielstruktur, können jedoch dann ohne Beschädigung des generierten Gewebes leicht von dieser entfernt werden.

Vorteilhafte Ausgestaltungen der Erfindung ergeben sich aus den in den Unteransprüchen genannten Merkmalen.

Die Erfindung wird nachfolgend in Ausführungsbeispielen anhand der zugehörigen Zeichnungen näher erläutert. Es zeigen:
- Figur 1: REM-Aufnahmen von Querschnitten (obere und mittlere Zeile) und Oberflächen (untere Zeile) von PAN- und P(AN-APMA)-Membranen;
- Figur 2: Ergebnisse eines LDH-Assays zur Untersuchung der Proliferation von HaCaT-Zellen auf PAN-Membranen sowie auf unmodifizierten, schwach modifizierten und stark modifizierten PEI-Membranen (PEI0, PEI1 bzw. PEI2);
- Figur 3: Laser-scanning-mikroskopische Aufnahmen von Vitalfärbungen der HaCaT-Zellen auf unmodifizierten, schwach modifizierten und stark modifizierten PEI-Membranen (PEI0, PEI1 bzw. PEI2);
- Figur 4: Mikroskopische Aufnahmen von H&E-Färbungen von Kryoschnitten organotypisch kokultivierter Keratinozyten auf P(AN-APMA)- und P(AN-NVP)-Membranen;
- Figur 5: Mikroskopische Aufnahmen von Immunofluoreszenzfärbungen der kokultivierten Keratinozyten auf P(AN-APMA)-Membran.

### Beispiel 1: Synthese von PAN und P(AN-APMA)

Die Acrylnitrilpolymere PAN (Polyacrylnitril, Homopolymer) gemäß Formel 1 sowie P(AN-APMA) (Polyacrylnitril-3-aminopropylmethacrylamid Hydrochlorid, Copolymer) gemäß Formel 2 wurden durch radikalinitiierte 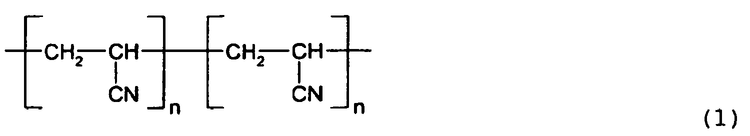 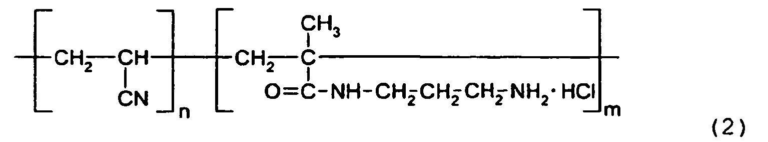 Lösungspolymerisation der entsprechenden Monomere und gegebenenfalls Comonomere (Acrylnitril bzw. Acrylnitril und 3-Aminopropylmethacrylamid) hergestellt, in Ethanol ausgefällt und getrocknet. Mittels Elementaranalyse wurden die Polymere hinsichtlich ihrer chemischen Zusammensetzung charakterisiert und durch Membranosmometrie ihre Molmasse bestimmt. Die Ergebnisse sind in Tabelle 1 zusammengestellt.

**Tabelle 1: Polymereigenschaften**

| Membran | X_{com} [Mol-%] | M_{n,OBm} [10³ g/mol] |
|---|---|---|
| PAN | 0 | 48,5 |
| P(AN-APMA) | 7,1 | 55,7 |

| | | |
|---|---|---|
| X_{com}= Molanteil des Comonomers im Polymer, M_{n,Obm}= Molekularmasse des Polymers. | | |

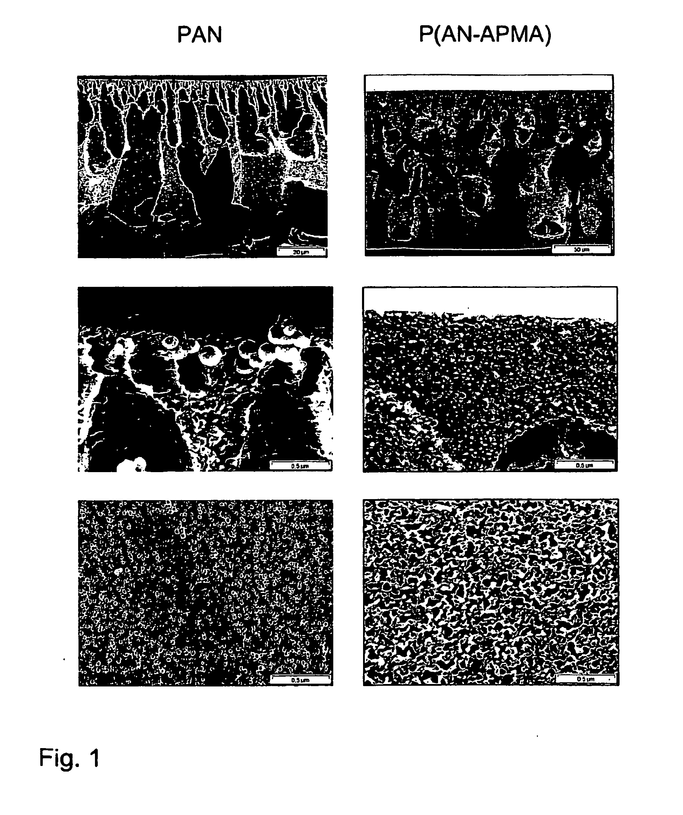

Aus den synthetisierten Polymeren wurden Membranen aus den entsprechenden DMF-Polymerlösungen durch Phaseninversionstechnik unter Einwirkung von Wasser hergestellt. Die Membranen wurden anschließend lösungsmittelfrei gewaschen und getempert. Einige der Membranen wurden zur besseren Handhabung auf ein Stützvlies (Histar TH100) gezogen. Bis zu ihrer Verwendung wurden die Membranen in saurer Lösung im Kühlschrank bei 4°C aufbewahrt. Charakteristische Daten zur Herstellung der Membranen und deren Eigenschaften sind in Tabelle 2 zusammengestellt. Figur 1 zeigt rasterelektronenmikroskopische Aufnahmen der Querschnitte (obere und mittlere Zeile) und der Oberflächen (untere Zeile) der PAN- und P(AN-APMA)-Membranen.

**Tabelle 2: Herstellungsparameter**

| Membran | C_{Polymer} [Gew.-%] | V_{Zug} [m/min] | T_{Lsg} [°C] | Fällbad | Tempern |
|---|---|---|---|---|---|
| PAN | 15 | 4 | 1-2 | H₂O 5°C | 10 min 90°C |
| P(AN-APMA) | 15 | 2 | RT | H₂O RT | 10 min 90°C |

| | | | | | |
|---|---|---|---|---|---|
| C_{polymer}= Konzentration des Polymers in der Lösung, v_{Zug}= Zuggeschwindigkeit, T_{Lsg}= Temperatur der Polymerlösung. | | | | | |

### Beispiel 2: Herstellung aminierter PEI-Membranen

Polyetherimid-Membranen (PEI-Membranen) gemäß Formel 3 wurden durch Oberflächenfunktionalisierung kommerziell erhältlicher PEI-Membranen mit Ultrafiltrationseigenschaften mittels oligomerer oder polymerer, unverzweigter oder verzweigter Amine (insbesondere Polyethylenimine) entsprechend einem von Albrecht et al. beschriebenen Verfahren aminiert (Albrecht W, Seifert B, Weigel Th, Schossig M, Holländer A, Groth Th, Hilke R: Amination of polyetherimide membranes using di and multivalent amines, Macromol. Chem. Phys. 2003, 204, 510-521). Dabei erfolgt eine kovalente Aminierung der Carbonylgruppen des Imidringes der PEI-Membran durch das Polyethylenimin -[(CH₂)₂NH]ₙ- unter Ausbildung einer Amidbindung entsprechend Formel 4. In Abhängigkeit davon, ob die Reaktion mit der endständigen primären Aminogruppe oder mit einer sekundären Aminogruppe des Polyethylenimins erfolgt, liegt die gebildete Amidgruppe in Form von CONHR oder von CONR¹R² vor, wobei die Reste R, R¹ und R² jeweils einen unverzweigten oder verzweigten Polyethyleniminrest [(CH₂)₂NH]ₙ darstellen.

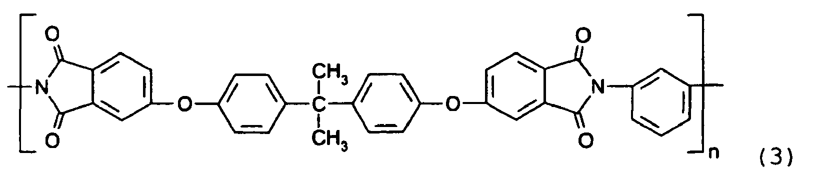

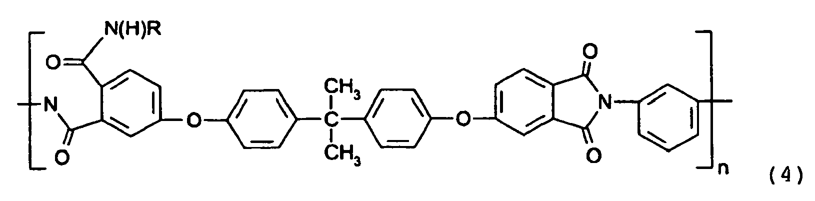

Im konkreten Beispiel wurden PEI-Ultrafiltrationsmembranen mit einem niedermolekularen, unverzweigten Polyethylenimin mit einer mittleren Molekularmasse von etwa 700 g/mol aminiert (PEI-Amin1) und mit einem hochmolekularen, verzweigten Polyethylenimin mit einer mittleren Molekularmasse von etwa 60.000 g/mol (PEI-Amin2). Dabei resultierte im Fall von PEI-Amin2 aufgrund der hohen Anzahl von Aminfunktionen innerhalb jeder Kette ein deutlich höherer Amingehalt. Charakteristische Daten der auf diese Weise oberflächenmodifizierten, aminierten PEI-Membranen sowie der gemäß Beispiel 1 hergestellten substratmodifizierten PAN- und P(AN-APMA)-Membranen sind in Tabelle 3 aufgelistet. Der Amingehalt wurde mit einem Azidorange-Nachweis bestimmt, der im Einzelnen in W. Albrecht et al. (s.o.) beschrieben ist.

**Tabelle 3: Membraneigenschaften**

| Membran | Wasser fluss [1/m²hkPa] | Kontaktwinkel [°] | | Amingehalt [nmol/cm²] |
|---|---|---|---|---|
| | | Benettzung | Entnetzung | |
| PAN | 4,02 | 59,5 | 43,0 | 0 |
| P(AN-APMA) | 0,70 | 81,3 | 48,4 | 400 |
| PEI | 3,86 | 79,5 | 48,5 | 0 |
| PEI-Amin1 | 3,5 | 61,8 | 33,8 | 410 |
| PEI-Amin2 | 0,7 | 55,8 | 13,2 | 650 |

### Beispiel 3: Bestimmung der Materialtoxizität

Vor Einsatz der gemäß Beispiel 1 und 2 hergestellten Trägermembranen in Zellbesiedlungsversuchen wurde die Toxizität der Materialien gemäß ISO 10993-5 untersucht. Dabei wurden Materialextrakte durch Inkubation mit Zellkulturmedium für den Zeitraum von 1, 3, 7 und 14 Tagen hergestellt. 3T3-Fibroblasten wurden in 96-Loch Kulturplatten bis zur Präkonfluenz, das heißt bis vor Erreichen der dichtest möglichen Anordnung von Zellen als Monolayer, kultiviert und dann für 24 Stunden mit den Extraktionsmedien überschichtet. Nachfolgend wurde die metabolische Aktivität und Membranintegrität der Zellen durch LDH- beziehungsweise XTT-Assays im Vergleich zu Kontrollmaterialien (3T3-Fibroblasten kultiviert in reinem Zellkulturmedium) bestimmt. In keinem Falle konnte eine toxische Wirkung nachgewiesen werden (Ergebnisse nicht dargestellt).

### Beispiel 4: Kultivierung epidermaler Hautstrukturen auf aminierten Membranen

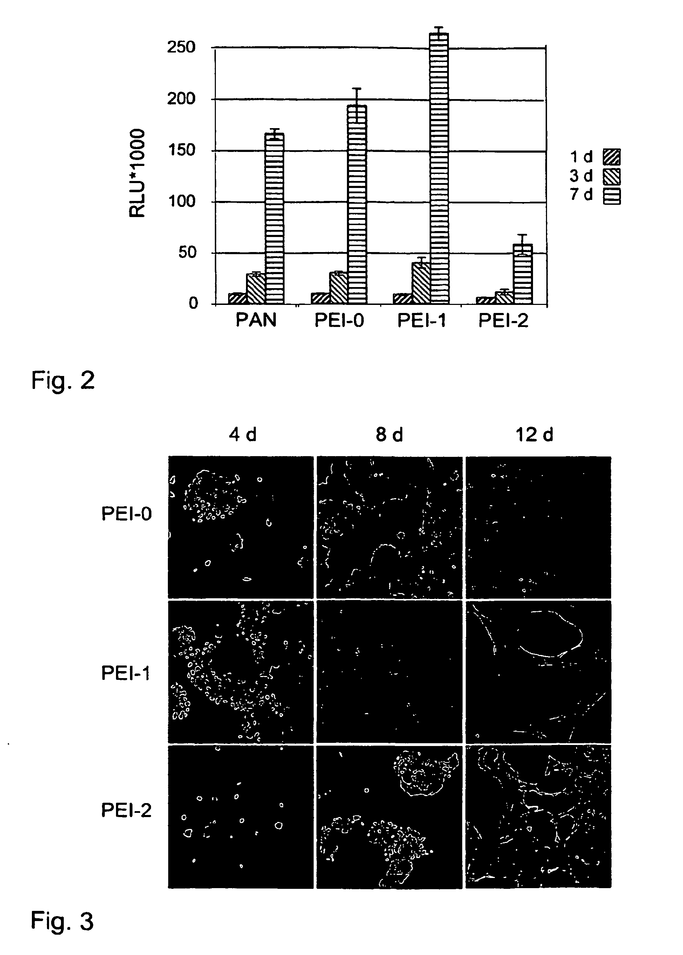

Die Keratinozyten-Zelllinie HaCaT wurde auf den Polymermembranen nach den Beispielen 1 und 2 PAN, PEI (=PEI-0), PEI-Amin1 (=PEI-1) und PEI-Amin2 (=PEI-2) ausgesät. Nach 1, 3 und 7 Tagen Inkubation im Brutschrank wurde die Aktivität der zellulären Laktatdehydrogenase in einem LDH-Assay bestimmt. Die Ergebnisse sind in Figur 2 dargestellt. Die weder substanz- noch oberflächenaminierten Membranen PAN und PEI-0 zeigten vergleichbare Aktivitäten. Die "moderat" aminierten PEI-Membranen PEI-1 mit einem Amingehalt von 410 nmol/cm² zeigten nach 7 Tagen die höchste Enzymaktivität. Demgegenüber wurde bei den stark aminierten Membranen (PEI-2) mit einem Amingehalt von 650 nmol/cm² eine deutliche Reduktion der Enzymaktivität gegenüber den nicht-aminierten Membranen festgestellt.

Bestätigt wurden diese Ergebnisse durch eine Vitalfärbung der HaCaT-Zellen mit Fluoresceindiacetat und anschließender Auswertung im Laser-Scanning-Mikroskop (Figur 3). Schon nach 8 Tagen bildeten die HaCaTZellen auf der schwach aminierten PEI-1-Membran einen geschlossenen Zellrasen aus. Bemerkenswert sind die Resultate nach 12 Tagen auf der PEI-1-Membran, wonach die Zellen sich großflächig von der Polymermembran ablösen. LDH-Messungen haben jedoch ergeben, dass die Zellen eine sehr hohe Enzymaktivität behalten. Dies lässt den Schluss zu, dass die aminierte Polymermembran das Zellwachstum von Keratinozyten stimuliert, die Zellen auf dem Material wunschgemäß jedoch nur schwach adhärieren.

### Beispiel 5: Kultivierung epidermaler Hautstrukturen auf Acrylnitrilcopolymer-Membranen

Zu diesen Versuchen wurden gemäß Beispiel 1 hergestellte UF-Membranen aus P(AN-APMA) sowie eine analog Beispiel 1 hergestellte UF-Membran aus dem Copolymer Polyacrylnitril-N-vinyl-pyrrolidon (P(AN-NVP)) gemäß Formel 5 eingesetzt. Beide Materialien zeichnen sich gemäß Kontaktwinkelmessung durch eine ähnliche Hydrophilie aus.

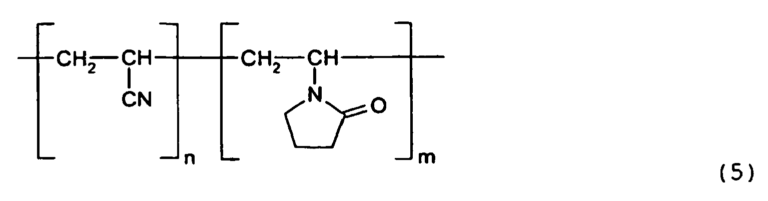

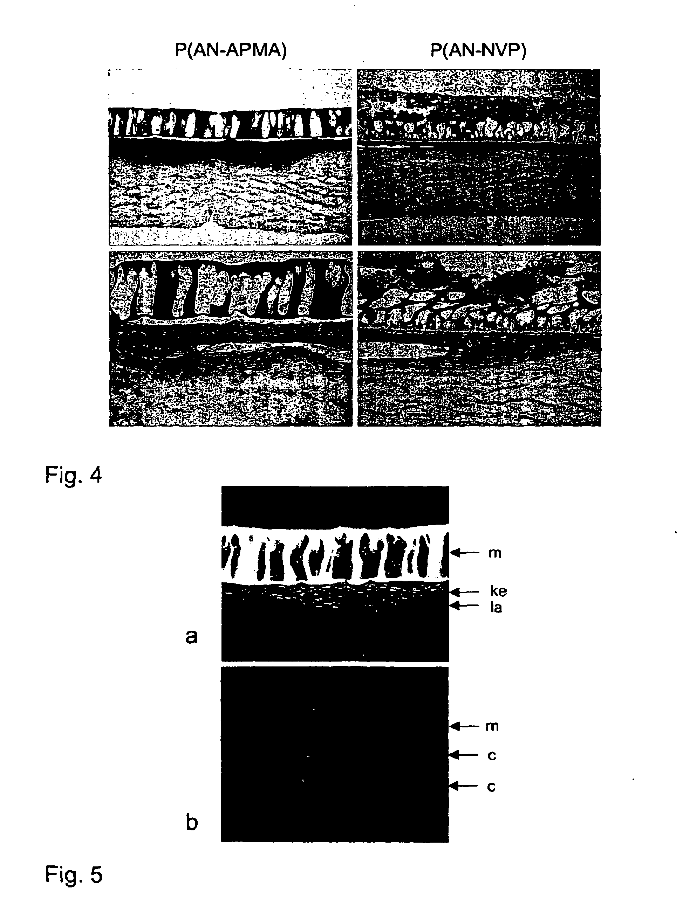

Primäre Keratinozyten wurden auf diesen UF-Membranen ausgesät und nach 6 Tagen Kultivierung mit ihren Zellseiten ("upside down") auf ein präformiertes standardisiertes Dermisäquivalent, bestehend aus Fibroblasten in einem Kollagengel, gelegt. Zu diesem Zeitpunkt bedeckten die Zellen die UF-Membranen nahezu vollständig. Nach weiteren 6 Tagen in serumhaltigem Medium und unter Luftexposition der Keratinozyten wurden diese organotypischen Kokulturen histologisch untersucht. Abhängig vom Material der Membranen zeigten sich unterschiedliche Ergebnisse, wie anhand der in Figur 4 gezeigten H&E-Färbung von Kryoschnitten ersichtlich ist (obere und untere Reihe: 10-fache bzw. 20-fache Vergrößerung). Unter den P(AN-NVP)-Membranen (Figur 4, rechte Seite) bildeten sich nur sehr dünne Neoepidermisstrukturen, die stark adhärent mit der Materialoberfläche verbunden blieben. Hingegen hatte sich die deutlich stärker ausgebildete Epidermis unter der P(AN-APMA)-Membran (Figur 4, linke Seite) spontan nahezu vollständig von dieser aminierten Oberfläche abgelöst. Hierdurch ist besonders vorteilhaft eine einfache Entfernung der UF-Membran ohne Beschädigung des neu gebildeten Gewebes gewährleistet.

Es wird vermutet, dass die leichtere Ablösung der Hautstruktur von der P(AN-APMA)-Membran im Zusammenhang mit dem Vorliegen primärer und sekundärer Aminogruppen beziehungsweise von stickstoffgebundenen Protonen des Membranmaterials steht.

Die auf der P(AN-APMA)-Membran *in vitro* gebildete Hautkultur wurde genauer mittels immunofluoreszenzmikroskopischer Analyse untersucht. In Figur 5a ist die im Original grün fluoreszierende Immunofluoreszenzfärbung von Keratin 1/10 (ke) gezeigt sowie die original rote Fluoreszenzfärbung von Laminin (la). Figur 5b zeigt eine Immunofluoreszenzfärbung mit dem Thymidinanalogon Bromdesoxyuridin (BrdU), bei dem die Zellkerne (c) im Original eine blaue Fluoreszenzfärbung erhalten. Die P(AN-APMA)-Membranen (m) zeigen in beiden Darstellungen eine Autofluoreszenz. Mit dieser Untersuchung ließen sich alle Merkmale einer gesunden Haut wiederfinden. Differenzierende Keratinozyten verstärken das Zytoskelett der Haut durch Synthese von Keratin 1 und Keratin 10 und zwischen Dermis und Epidermis bildet sich die Basalmembran mit einem hohen Anteil an Laminin-5 (Figur 5a). Überraschenderweise wurden proliferierende, BrdU-positive Keratinozyten nicht nur angrenzend an die Basalmembran gefunden, sondern ebenso in dem Epithelanteil, der in Kontakt mit der aminierten UF-Membran steht (Figur 5b). Diese doppelseitige Polarisierung des neu gebildeten Epithels ist dadurch bedingt, dass ein Teil der Zellen auf dem Trägermaterial zum Zeitpunkt der Umpflanzung auf das Dermisäquivalent bereits differenziert war und sich nachfolgend nicht umorientieren konnte. Dies lässt sich durch eine Verkürzung der Anzüchtungszeit auf dem Trägermaterial und eine frühzeitige Umorientierung, das heißt Umdrehen des Trägermaterials, verhindern, so dass die darauf wachsenden Keratinozyten frühzeitig mit ihrer Oberseite mit dem zu besiedelnden Wundbett in Kontakt kommen.

## Patentansprüche

1. Verwendung eines polymeren Substrats zur Kultivierung von Keratinozyten, wobei das Substrat in Form einer sauerstoffdurchlässigen Flachmembran aus einem nicht resorbierbaren Polymer vorliegt und die Membran einen Amingehalt von mindestens 10 nmol Aminfunktionen pro cm² Membran aufweist, wobei die Polymersubstanz der Membran ein Copolymer aus Acrylnitril (AN) und 3-Amino-propylmethacrylamid (APMA) umfasst oder die Membran eine mit einem Polyethylenimin aminierte Polyetherimid-Membran ist.

2. Verwendung nach Anspruch 1, **dadurch gekennzeichnet,**
**dass** die Membran einen Amingehalt von mindestens 50, insbesondere von 100 bis 550, insbesondere von 200 bis 500, vorzugsweise von etwa 350 bis 450 nmol Aminfunktionen pro cm² Membran aufweist.

3. Verwendung nach einem der Ansprüche 1 oder 2, **dadurch gekennzeichnet,**
**dass** der Amingehalt Stickstofffunktionen, in denen der Stickstoff mindestens eine Wasserstoffbindung aufweist, insbesondere primäre und/oder sekundäre Aminfunktionen, betrifft.

4. Verwendung nach einem der vorhergehenden Ansprüche, **dadurch gekennzeichnet,**
**dass** die Membran eine für Mikroorganismen, insbesondere für Bakterien, undurchlässige Ultrafiltrationsmembran ist.

5. Verwendung nach einem der vorhergehenden Ansprüche, **dadurch gekennzeichnet,**
**dass** die Membran eine Porengröße von höchstens 0,5 µm, insbesondere höchstens 0,1 µm aufweist.

6. Verwendung nach einem der vorhergehenden Ansprüche, **dadurch gekennzeichnet,**
**dass** die Membran eine Wasserdurchlässigkeit von mindestens 0,5 l/m²hPa, insbesondere von mindestens 1,0 l/m²hPa aufweist.

7. Verwendung nach einem der vorhergehenden Ansprüche, **dadurch gekennzeichnet,**
**dass** die Membran eine plastische Oberflächenstrukturierung aufweist.

8. Verwendung nach Anspruch 7, **dadurch gekennzeichnet, dass** die plastische Oberflächenstrukturierung durch nachträgliche Prägung der Membran oder bereits während der Herstellung der Membran erzeugt ist.

9. Verwendung nach einem der vorhergehenden Ansprüche zur Erzeugung einer epidermalen Hautstruktur.

10. Organotypische Hybridstruktur umfassend
(a) ein polymeres Substrat, das in Form einer sauerstoffdurchlässigen Flachmembran aus einem nicht resorbierbarem Polymer vorliegt und die Membran einen Amingehalt von mindestens 10 nmol Aminfunktionen pro cm² Membran aufweist, wobei die Polymersubstanz der Membran ein Copolymer aus Acrylnitril (AN) und 3-Aminopropylmethacrylamid (APMA) umfasst oder die Membran eine mit einem Polyethylenimin aminierte Polyetherimid-Membran ist, sowie
(b) auf dem Substrat kultivierte Keratinozyten oder eine epidermale Hautstruktur.

11. In vitro Verfahren zur Erzeugung einer epidermalen Hautstruktur mit den Schritten:
- Kultivierung von Keratinozyten über eine erste Kultivierungsdauer auf einem polymeren Substrat, wobei das Substrat in Form einer sauerstoffdurchlässigen Flachmembran aus einem nicht resorbierbaren Polymer vorliegt und die Membran einen Amingehalt von mindestens 50 nmol Amin pro cm² Membran aufweist, wobei die Polymersubstanz der Membran ein Copolymer aus Acrylnitril (AN) und 3-Aminopropylmethacrylamid (APMA) umfasst oder die Membran eine mit einem Polyethylenimin aminierte Polyetherimid-Membran ist,
- Transfer des mit den Zellen besiedelten Substrats auf eine Zielstruktur, so dass das Substrat mit seiner zellzugewandten Seite auf der Zielstruktur aufliegt,
- Kultivierung der Zellen für eine zweite Kultivierungsdauer und
- Ablösung und Entfernen des Substrats von der gebildeten Hautstruktur.

## Claims

1. Use of a polymer substrate for culturing keratinocytes, wherein the substrate is an oxygen-permeable flat membrane made from a non-resorbable polymer and the membrane has an amine content of at least 10 nmol of amine functions per cm² of membrane, wherein the polymeric substance of the membrane comprises a copolymer of acrylic nitrile (AN) and 3-aminopropylmethacrylamide (APMA) or the membrane is a polyethylenimine-aminated polyetherimide membrane.

2. Use according to Claim 1, **characterized in that** the membrane has an amine content of at least 50, in particular from 100 to 550, in particular from 200 to 500, preferably from about 350 to 450, nmol of amine functions per cm² of membrane.

3. Use according to either of Claims 1 and 2, **characterized in that** the amine content relates to nitrogen functions in which the nitrogen has at least one hydrogen bond, in particular primary and/or secondary amine functions.

4. Use according to any of the preceding claims, **characterized in that** the membrane is an ultrafiltration membrane which is impermeable for microorganisms, in particular for bacteria.

5. Use according to any of the preceding claims, **characterized in that** the membrane has a pore size of no more than 0.5 µm, in particular no more than 0.1 µm.

6. Use according to any of the preceding claims, **characterized in that** the membrane has a water permeability to at least 0.5 l/m²hPa, in particular to at least 1.0 l/m²hPa.

7. Use according to any of the preceding claims, **characterized in that** the membrane has a three-dimensional surface structure.

8. Use according to Claim 7, **characterized in that** the three-dimensional surface structure has been generated by subsequent imprinting of the membrane or already during preparation of the membrane.

9. Use according to any of the preceding claims for generating an epidermal skin structure.

10. Organotypical hybrid structure comprising
(a) a polymer substrate which is an oxygen-permeable flat membrane made from a non-resorbable polymer and the membrane has an amine content of at least 10 nmol of amine functions per cm² of membrane, wherein the polymeric substance of the membrane comprises a co-polymer of acrylic nitrile (AN) and 3-aminopropylmethacrylamide (APMA) or the membrane is a polyethylenimine-aminated polyetherimide membrane, and
(b) keratinocytes cultured on the substrate or an epidermal skin structure.

11. In-vitro method for generating an epidermal skin structure, comprising the steps of
- culturing keratinocytes on a polymer substrate over a first cultivation period, wherein the substrate is an oxygen-permeable flat membrane made from a non-resorbable polymer and the membrane has an amine content of at least 50 nmol of amine per cm² of membrane, wherein the polymeric substance of the membrane comprises a co-polymer of acrylic nitrile (AN) and 3-aminopropylmethacrylamide (APMA) or the membrane is a polyethylenimine-aminated polyetherimide membrane,
- transferring the substrate colonized by the cells to a target structure such that the substrate rests on the target structure with its cell-facing side,
- culturing the cells for a second cultivation period, and
- detaching and removing the substrate from the skin structure formed.

## Revendications

1. Utilisation d'un substrat polymère pour la culture de kératinocytes, où le substrat se trouve sous forme d'une membrane plane perméable à l'oxygène constituée par un polymère non résorbable et la membrane présente une teneur en amine d'au moins 10 nmoles de fonctions amine par cm² de membrane, où la substance polymère de la membrane comprend un copolymère d'acrylonitrile (AN) et de 3-aminopropylméthacrylamide (APMA) ou la membrane est une membrane de polyétherimide aminé avec une polyéthylène-imine.

2. Utilisation selon la revendication 1, **caractérisée en ce que** la membrane présente une teneur en amine d'au moins 50, en particulier de 100 à 550, en particulier de 200 à 500, de préférence d'environ 350 à 450 nmoles de fonctions amine par cm² de membrane.

3. Utilisation selon l'une quelconque des revendications 1 ou 2, **caractérisée en ce que** la teneur en amine présente des fonctions azotées, dans lesquelles l'azote présente au moins une liaison hydrogène, en particulier des fonctions amine primaires et/ou secondaires.

4. Utilisation selon l'une quelconque des revendications précédentes, **caractérisée en ce que** la membrane est une membrane d'ultrafiltration imperméable aux microorganismes, en particulier aux bactéries.

5. Utilisation selon l'une quelconque des revendications précédentes, **caractérisée en ce que** la membrane présente une taille de pores d'au plus 0,5 µm, en particulier d'au plus 0,1 µm.

6. Utilisation selon l'une quelconque des revendications précédentes, **caractérisée en ce que** la membrane présente une perméabilité à l'eau d'au moins 0,5 l/m²hPa, en particulier d'au moins 1,0 l/m²hPa.

7. Utilisation selon l'une quelconque des revendications précédentes, **caractérisée en ce que** la membrane présente une structure de surface plastique.

8. Utilisation selon la revendication 7, **caractérisée en ce que** la structure de surface plastique est produite par gaufrage ultérieur de la membrane ou déjà pendant la fabrication de la membrane.

9. Utilisation selon l'une quelconque des revendications précédentes pour la production d'une structure de peau épidermique.

10. Structure hybride organotypique, comprenant
(a) un substrat polymère, qui se trouve sous forme d'une membrane plane perméable à l'oxygène en un polymère non résorbable et la membrane présente une teneur en amine d'au moins 10 nmoles de fonctions amine par cm² de membrane, où la substance polymère de la membrane comprend un copolymère d'acrylonitrile (AN) et de 3-aminopropylméthacrylamide (APMA) ou la membrane est une membrane de polyétherimide aminé avec une polyéthylène-imine, ainsi que
(b) des kératinocytes ou une structure de peau épidermique cultivés sur le substrat.

11. Procédé in vitro pour la production d'une structure de peau épidermique présentant les étapes :
- culture de kératinocytes pendant une première durée de culture sur un substrat polymère, où le substrat se trouve sous forme d'une membrane plane perméable à l'oxygène en un polymère non résorbable et la membrane présente une teneur en amine d'au moins 50 nmoles d'amine par cm² de membrane, où la substance polymère de la membrane comprend un copolymère d'acrylonitrile (AN) et de 3-aminopropylméthacrylamide (APMA) ou la membrane est une membrane de polyétherimide aminé avec une polyéthylène-imine,
- transfert du substrat ensemencé par les cellules sur une structure cible, de manière telle que le substrat se trouve avec sa face orientée vers les cellules sur la structure cible,
- culture des cellules pour une deuxième durée de culture et
- décollement et élimination du substrat de la structure de peau formée.
